Europäisches Patentamt

European Patent Office

Office européen des brevets

(19)

(11) Veröffentlichungsnummer: **0 271 908**
**A2**

(12) **EUROPÄISCHE PATENTANMELDUNG**

(21) Anmeldenummer: **87118723.3**

(22) Anmeldetag: **17.12.87**

(51) Int. Cl.4: **C07K 3/18** , **C07K 15/06** ,
**C07K 15/00** , **A61K 35/16** ,
**A61K 37/02**

(30) Priorität: **17.12.86 HU 525486**

(43) Veröffentlichungstag der Anmeldung:
**22.06.88 Patentblatt 88/25**

(84) Benannte Vertragsstaaten:
**AT BE CH DE FR GB IT LI NL SE**

(71) Anmelder: **Richter Gedeon Vegyészeti Gyár R.T.**
**Gyömröi ut 19-21**
**H-1475 Budapest X(HU)**

(72) Erfinder: **Knoll, Jozsef, Dr.**
**Jaszai Mari tér 4 b**
**H-1137 Budapest(HU)**
Erfinder: **Harmath, Sandor, Dr.**
**Tavirozsa u. 9**
**H-1161 Budapest(HU)**
Erfinder: **Nagy, Janos, Dr. Dipl.-Chem.**
**Jokai u. 9**
**H-2000 Szentendre(HU)**
Erfinder: **Knoll, Berta, Dr.**
**Madach l. u. 2-6**
**H-1075 Budapest(HU)**

(74) Vertreter: **Hansen, Bernd, Dr.rer.nat. et al**
**Hoffmann, Eitle & Partner Patentanwälte**
**Arabellastrasse 4**
**D-8000 München 81(DE)**

(54) **Appetitregulierende gereinigte Wirkstoffe biologischen Ursprungs, ihre Antikörper und die mit den entsprechenden Antikörpern gebildeten Immunkomplexe der Wirkstoffe.**

(57) Die stark appetitzügelnden Wirkstoffe Satietin und Satietin-D wurden aus menschlichem oder tierischem Blutserum oder Blutplasma bisher durch Ultrafiltration, Gelchromatographie, einer Behandlung mit Trichloressigsäure, Affinitätschromatographie und fallweise einer Behandlung mit einem proeolytischen Enzym hergestellt.

Erfindungsgemäß werden nun mit einem derartigen Präparat lebende Organismen, zum Beispiel Kaninchen, immunisiert. Da das Satietin bzw. Satietin-D Glycoproteine sind, bilden sich in dem lebenden Organismus Antikörper (Antisatietin und Antisatietin-D). Diese Antikörper können aus dem Blut des lebenden Organismus isoliert und an eine Gelsäule gebunden werden. Zur Herstellung von Satietin bzw. Satietin-D wird nun menschliches oder tierisches Blutserum oder Blutplasma ultrafiltriert, und das Ultrafiltrat - das außer Satietin noch Serumeiweiße und sonstige Bestandteile geringeren Molekulargewichtes enthält - wird mit der Gelsäule kontaktiert. Der an die Gelsäule gebundene Antikörper bildet mit dem Satietin nun einen Immunkomplex, der auf der Säule bleibt, während die übrigen Bestandteile des Ultrafiltrates mit Puffer von der Säule heruntergewaschen werden können. Dann wird der Immunkomplex mit einem geeigneten Agens zerlegt; das Satietin kann in reiner Form von der Säule eluiert werden, die den Antikörper enthaltende Säule ist erneut verwendbar.

## Appetitregulierende gereinigte Wirkstoffe biologischen Ursprungs, ihre Antikörper und die mit den entsprechenden Antikörpern gebildeten Immunkomplexe der Wirkstoffe

Die Erfindung betrifft selektiv appetitgerulierende gereinigte Wirkstoffe biologischen Ursprungs, nämlich die Wirkstoffe Satietin und Satietin-D in gereinigter Form; sie werden mit einer Immunabsorbensmethode gewonnen.

Die Erfindung betrifft ferner den Satietin-Antikörper (im folgenden Antisatietin) und den Satietin-D-Antikörper (im folgenden Antisatietin-D), die die determinanten Gruppen des Satietins beziehungsweise Satietin-D erkennen.

Antisatietin und Antisatietin-D werden zweckmäßig hergestellt, indem man Satietin beziehungsweise Satietin-D in üblicherweise für Immunisierungszwecke verwendete lebende Organismen parenteral einbringt und nach Ablauf der entsprechenden Zeit das Antisatietin beziehungsweise das Antisatietin-D aus dem Antiserum entsprechender Spezifität und Avidität in bekannter Weise abtrennt.

Schließlich betrifft die Erfindung die von Satietin mit Antisatietin beziehungsweise die von Satietin-D mit Antisatietin-D gebildeten Immunkomplexe, für deren Herstellung charakteristisch ist, daß man Satietin mit Antisatietin beziehungsweise Satietin-D mit Antisatietin-D unter Bedingungen, die die Bindung ermöglichen, kontaktiert.

Die erfindungsgemäßen Immunkomplexe werden an einer festen Phase, vorzugsweise einer Gelsäule, insbesondere einer aktivierten und äquilibrierten Gelsäule, gebildet. Aus dem Immunkomplex werden Satietin beziehungsweise Satietin-D mit einem zum Spalten von Immunkomplexen geeigneten Agens freigesetzt. Auf diese Weise können Satietin beziehungsweise Satietin-D in reiner, nativer Form hergestellt werden.

Die Erfindung betrifft auch ein Verfahren zur Herstellung von Satietin und Satietin-D in gereinigter Form aus menschlichem oder tierischem Blutserum oder Blutplasma.

Für das Verfahren ist kennzeichnend, daß man menschliches oder tierisches Blutserum oder Blutplasma einer Untralfiltration, einer Gelchromatographie, einer Behandlung mit Trichloressigsäure, einer Affinitätschromatographie und gewünschtenfalls der Behandlung mit einem proteolytischen Enzym, vorzugsweise Trypsin und Chymotrypsin, unterzieht, die Reinheit und Homogenität des erhaltenen Produktes mit einer immunchemischen Methode, vorzugsweise durch Immunoelektrophorese, kontrolliert, zu dem immunchemisch homogenen Präparat Adjuvant gibt und das adjuvierte Präparat parenteral einem für die Zwecke der Immunisierung üblichen lebenden Organismus verabreicht und nach Ablauf der entsprechenden Zeit das polyclone monovalente Anti satietin und Antisatietin-D aus dem aus dem Blut des lebenden Organismus erhaltenen Antiserum geeigneter Spezifität und Avidität abtrennt und an eine geeignete feste Phase, vorzugsweise an ein aktiviertes und äquilibriertes Gel, bindet, dann durch die die Antikörper enthaltende Gelsäule ein äquilibriertes Ultrafiltrat, das die natürlichen, ein Molgewicht von weniger als 50 000 Dalton aufweisenden Bestandteile des menschlichen oder tierischen Blutserums oder Blutplasmas enthält, hindurchlaufen läßt und dann von der Säule aus dem von Satietin und Antisatietin beziehungsweise aus dem von Satietin-D und Antisatietin-D gebildeten Immunkomplex das Satietin beziehungsweise das Satietin-D selektiv desorbiert, die Eluate sammelt und gewünschtenfalls lyophilisiert.

Satietin und Satietin-D sind spezifische auf das Appetitszentrum wirkende, appetitzügelnde Wirkstoffe.

In der britischen Patentschrift Nr. 2 056 993 ist ein Verfahren beschrieben, mit dem aus menschlichem oder tierischem Blutserum ein neuartiger, selektiv wirkender appetitzügelnder Wirkstoff hergestellt werden kann. Diese aus Serum und Plasma durch Ultrafiltration und Gelchromatographie hergestellte, mit dem Phantasienamen Satietin bezeichnete Substanz wird im Interesse einer weiteren Reinigung gemäß der US-PS 4 430 264 weiteren chemischen Eingriffen unterzogen. So wurde durch Behandeln mit Trichloressigsäure und Affinitätschromatographie die Aktivität des Wirkstoffes weiter erhöht. Im Zuge der Weiterentwicklung der Erfindung wurde das Verfahren durch neue Elemente ergänzt (siehe US-PS 4 588 685), die es ermöglichten, eine weitere, selektiv appatitzügelnde, chemisch einheitliche und in ihren physikalischen Eigenschaften von Satietin abweichende Substanz zu isolieren, die den Phantasie namen Satietin-D erhielt. Dies geschieht, indem das auf die beschriebene Weise durch Ultrafiltration und Gelchromatographie sowie Behandeln mit Trichloressigsäure und Affinitätschromatographie hergestellte Produkt durch Behandeln mit Trypsin und Chymotrypsin weiter gereinigt wird. Diese Methoe beruht auf der Erkenntnis, daß das Satietin ein Glycoprotein ist und einer Behandlung mit proteolytischen Enzymen widersteht und seine biologische Aktivität bewahrt, während die begleitenden Eiweißverunreinigungen durch die Enzyme zerstört werden und entfernt werden können.

Im Zuge der Weiterentwicklung der beschriebenen Erfindung wurde nun gefunden, daß die Isolierung der einen natürlichen Bestandteil des menschlichen und tierischen Blutes bildenden Substanzen Satietin und Satietin-D vereinfacht und spezifischer gemacht werden kann, wenn man das Satietin oder das Satietin-D aus dem Ultrafiltrat des menschlichen oder trierischen Blutserums oder Blutplasmas mittels einer spezifischen Variante der Affinitätschromatographie, und zwar mittels der Immunoadsorbens-Chromatographie, isoliert.

Im folgenden wird das erfindungsgemäße Verfahren ausführlicher beschrieben.

1. Gemäß der britischen Patentschrift Nr. 2 056 993 beziehungsweise den US-PS 4 430 264 und 4 588 685 werden Satietin-beziehungsweise Satietin-D-Präparate hergestellt. Deren Reinheit und Homogenität wird außer mit den bereits bekannten Methoden auch mit immunchemischen Methoden, vorzugsweise mittels Immunoelektrophorese [Int. Arch. All. Appl. Immunol. 7, 103 (1955)], zweidimensionaler Immunoelektrophorese [Anal. Biochem. 10, 358 (1965), Clin. Scien. 35 (5), 403 (1968)] geprüft, wobei die menschlichen Serumeiweiße ausfällende polyvalente Anti körper beziehungsweise bekannte Serumeinweiße ausfällende monovalente Antikörper verwendet werden. Auf diese Weise können eventuell vorhandene Eiweiße identifiziert beziehungsweise kann die Reinheit der Präparate festgestellt werden. Wenn das Präparat immunchemisch homogen ist, wird es gepuffert und dann adjuviert, vorzugsweise mit einem inkompletten Freund-Adjuvant, und dann parenteral in einen für Immunisierungszwecke üblicherweise eingesetzten lebenden Organismus, vorzugsweise Kaninchen oder Ziegen, einbringt. Zweckmäßig werden die Tiere mehrmals geimpft. Ziel ist die Herstellung polycloner monovalenter Antikörper gegen Satietin beziehungsweise Satietin-D. Nach entsprechender Zeit, wenn in den Blutproben Antiserum mit entsprechender Avidität und entsprechenden Fällungseigenschaften nachweisbar ist, werden die Tiere getötet und ausgeblutet. Das Blut wird zentrifugiert. Aus dem Überstand, d.h. aus dem Antiserum, werden die Antikörper auf die übliche Weise, zum Beispiel durch Gelfiltration und/oder präparatuve Elektrophorese und/oder Aussalzen und/oder Ionenaustauscherchromatographie, vorzugsweise durch Aussalzen und Ionenaustauschchromatographie isoliert.

2. Das auf die unter 1. beschriebene Weise hergestellte polyclon-monovalente Antisatietin beziehungsweise Antisatietin-D wird an eine feste Phase, vorzugsweise an aktiviertes und äquilibriertes Gel, insbesondere an Sepharose-Gel gebunden und aus dem Gel dann eine Gelsäule geformt. Als Aktivierungsmittel wird vorzugsweise Cyanbromid verwendet. Zum Äquilibrieren wird ein Puffer verwendet, dessen pH zwischen 2,5 und 10 liegt. Geeignete Puffer sind zum Beispiel tris-Puffer [Gemisch aus tris-(Hydroxymethyl)aminomethan und Salzsäure; pH 8,5] oder Glycin-Salzsäure-Puffer (pH 2,5) oder Boratpuffer (0,025 M wässrige Lösung, pH: 8,8).

Die vorher berechnete Menge menschlichen oder tierischen Blutserums oder Plasmas wird durch ein Membranfilter filtriert, das Moleküle bis zu einer Größe von 50 000 Dalton durchläßt (für Satietin zum Beispiel Amicon UM 10, für die Herstellung von Satietin-D zum Beispiel Amicon hollow fiber $H_{10}P_{10}$). Das Ultrafiltrat, das alle natürlichen Bestandteile des Blutserums oder Blutplasmas enthält, deren Molekulargewicht unter 50 000 liegt, wird partiell, auf etwa ein Fünftel seines Volumens eingedickt. Das eingedickte Ultrafiltrat wird nun mit dem gleichen Puffer äquilibriert, der vorher zum Äquilibrieren der Gelsäule verwendet wurde. Das äquilibrierte Ultrafiltrat wird nun durch die den Antikörper enthaltende Gelsäule hindurchgelassen.

Das an das Gel fixierte Antisatietin beziehungsweise Antisatietin-D bindet den Satietin-beziehungsweise Satietin-D-Gehalt des Ultrafiltrats, und nur diesen. Die übrigen Bestandteile des Blutserums beziehungsweise Blutplasmas, die kleiner als 50 000 Dalton sind, können mit dem gleichen Puffer, der zum Äquilibrieren verwendet wurde, von der Säule heruntergewaschen werden. Nachdem eiweißfreies Eluat erscheint (das wird UV-spektrophotometrisch kontrolliert), wird die Säule mit einer zur Zersetzung von Immunkomplexen geeigneten Lösung durchgewaschen. Geeignet sind zum Beispiel die wässrigen Lösungen von Guanidinhydrochlorid, Harnstoff oder Ammoniumrhodanid. Letzteres ist bevorzugt. Diese Lösung zerstört die Bindung zwischen Satietin und Antisatietin beziehungsweise Satietin-D und Antisatietin-D, und auf diese Weise können Satietin beziehungsweise Satietin-D selektiv von der Säule desorbiert werden. Die Fraktionen werden gesammelt, durch Dialyse gegen destilliertes Wasser entsalzen und dann gewünschtenfalls lyophilisiert. Die biologische Aktivität des lyophilisierten Wirkstoffes wird auf die in der britischen Patentschrift Nr. 2 056 993 beschriebene Weise untersucht.

Das erfindungsgemäße Verfahren hat den Vorteil, die Abtrennung des nativen Satietins und Satietin-D aus dem Ultrafiltrat menschlichen oder tierischen Blutserums oder Blutplasmas wesentlich einfacher, mittels einer Immunabsorptionsmethode zu ermöglichen. Diese Methode ist schonender und spezifischer als die bisher angewendeten, und deshalb weist das Produkt - im Gegensatz zu den bischer hergestellten - die seinem natürlichen Ursprung entsprechenden Eigenschaften auf. Die biologische Aktivität ist die gleiche wie die der früher hergestellten Satietin und Satietin-D Präparate. Vor Beginn des erfindungsgemäßen Verfahrens muß praktisch nur einmal das zum Immunisieren erforderliche Präparat hergestellt werden, zum Beispiel nach einem der bekanneten Verfahren. Das erfindungsgemäße Verfahren ist sehr wirtschaftlich, denn die den Antikörper enthaltende Gelsäule kann sehr oft mit unverändertem Ergebnis verwendet werden. Die erfindungsgemäße Weiterentwicklung läßt sich auch in größere Maßstäbe umsetzen, das heißt, es können größere Mengen Ultrafiltrat aufgearbeitet werden.

Die Erfindung wird an Hand der folgenden Beispiele näher erläutert, ist jedoch nicht auf diese Beispiele beschränkt.

### Beispiel 1

Herstellung von gereinigtem Satietin

a) Mit einem Satietinpräparat (hergestellt gemäß der britischen Patentschrift Nr. 2 056 993 oder der US-PS 4 430 264) werden Kaninchen immunisiert, wobei das Präparat mit einem inkompletten Freund-Adjuvant adjuviert und dann in Reihenimpfungen appliziert. In bestimmten Zeitanstanden werden Blutproben genommen, ihre Spezifität und Avidität wird immunelektrophoretisch kontrolliert. Wenn das Antiserum genügend stark ist, läßt man die Tiere ausbluten. Das Blut wird zentrifugiert.

Der nach dem Zentrifugieren erhaltene Überstand ist das Antiserum. Zu 100 ml Antiserum werden unter ständigem Rühren 25 g festes Ammoniumsulfat gegeben. Das Gemisch wird eine Stunde lang stehen gelassen, dann wird der entstandene Niederschlag abzentrifugiert. Der Niederschlag wird mehrmals mit 1,75 M wässriger Ammoniumsulfatlösung gewaschen und erneut zentrifugiert. Der das Antisatietin enthaltende Niederschlag wird dann zuerst gegen destilliertes Wasser, dann bei +4 °C gegen 0,05 M wässrige Natriumacetatlösung dialysiert. Die Dialyse dauert einige Tage. Dabei entstehende Niederschlagsspuren werden durch Zentrifugieren von der Lösung abgetrennt.

Auf folgende Weise wird eine DEAE-Sephadex-Säule bereitet. Man läßt das trockene Gel in einer 0,05 M wässrigen Natriumacetatlösung quellen und bereitet dann daraus ein Gelbett geeigneter Größe. Das auf die oben beschriebene Weise hergestellte Dialysat wird auf das Gelbett aufgebracht. Die durch die Säule fließenden Fraktionen werden gesammelt, vereinigt und mit auf 100 ml Fraktionsvolumen gerechnet 25 g festem Ammoniumsulfat ausgesalzten. Der Niederschlag wird von der Flüssigkeit abgetrennt und gegen einen Phosphatpuffer des pH-Wertes 7,2 dialysiert. Das Dialysat wird eingeengt und gegen eine 0,1 M wässrige Sodalösung bei pH 8,8 und +4 °C erneut dialysiert.

b) 50 ml eines mit Cyanbromid aktivierten Gels (AH-Sepharose 4B) werden mit 0,1 M wässrigem Natriumcarbonatpuffer (pH 8,8) mehrmals durchgewaschen. Dann wird dem Gel eine 25 %ige Glutaraldehydlösung zugesetzt, wobei die Menge so bemessen wird, daß die Endkonzentration 2,5 % beträgt. Das Gel läßt man eine Stunde stehen. Dann wird die Glutaraldehydlösung durch Filtrieren auf einer Fritte entfernt und das Gel mit 0,1 M wässriger Sodalösung gewaschen (pH 8,8).

Nun wird das Gel mit dem gemäß a) hergestellten, das Antisatietin enthaltenden Dialysat vermischt und eine weitere Stunde lang bei 4 °C gerührt. Dann läßt man den Ansatz 16 Stunden lang stehen. Schließlich wird das Gel mit 0,1 M wässriger Sodalösung (pH 8,8) mehrmals durchgewaschen.

Als weitere Wäsche schließt sich eine mehrmalige Behandlung mit 0,2 M wässriger Äthanolaminlösung an, die dann durch erneutes Waschen mit 0,1 M wässriger Sodalösung entfernt wird. Dann wird das Gel mit 5 M wässriger Guanidinhydrochloridlösung gewaschen. Schließlich wird das Medium des Gels gegen 0,025 M Boratpuffer (pH 8,4) ausgewechselt.

Das Gel wird in eine Säule gefüllt und mit 0,025 M wässrigem Boratpuffer (pH 8,4) äquilibriert.

c) Menschliches Blutserum wird durch ein bis zu einem Molekulargewicht von 50 000 Dalten durchlässiges Membranfilter (Amicon UM 10) durchgelassen. Das erhaltene Ultrafiltrat wird gegen 0,025 M Boratpuffer (pH 8,4) dialysiert. Das erhaltene Dialysat wird auf die vorbereitete Gelsäule aufgebracht, und die von der Säule ablaufenden Fraktionen werden gesammelt. Das an das Gel gebundene Antisatietin bindet das Satietin aus dem Filtrat. Die nicht gebundenen sonstigen niedermolekularen Bestandteile des Serums, insbesondere die Serumeiweiße, können nun mit Boratpuffer (0,025 M, pH 8,4) von der Säule heruntergewaschen werden. Wenn das Eluat kein Eiweiß mehr enthält (Kontrolle mit UV-Spektrophotometrie), wird mit einer dem doppelten Gelvolumne entsprechenden Menge 3 M wässriger Ammoniumrhodanidlösung das Satietin aus dem an der Säule gebundenen Immunkomplex freigesetzt.

Das von der Säule desorbierte Satietin wird gegen Wasser dialysiert, um das Ammoniumrhodanid zu entfernen. Das Dialysat wird lyophilisiert. Die Aktivität des Lyophilisates wird auf die in der britischen Patentschrift Nr. 2 056 993 beschriebene Weise bestimmt.

### Beispiel 2

Herstellung von gereinigtem Satietin-D

Man arbeitet auf die im Beispiel 1 beschriebene Weise mit dem Unterschied, daß man im Schritt a) statt des Satietins ein Satietin-D-Präparat verwendet (das gemäß der US-PS 4 588 685 hergestellt ist) und im Schritt c) ein Membranfilter des Typs Amicon hollow fiber $H_{10}P_{10}$ verwendet.

Beispiel 3

Herstellung von gereinigtem Satietin

Man arbeitet auf die im Beispiel 1 beschriebene Weise mit dem Unterschied, daß man statt der Kaninchen Ziegen immunisiert.

Beispiel 4

Herstellung von gereinigtem Satietin-D

Man arbeitet auf die im Beispiel 2 angegebene Weise mit dem Unterschied, daß man statt der Kaninchen Ziegen immunisiert.

**Ansprüche**

1. Selektiv appetitzügelnde Wirkstoffe biologischen Ursprungs, Satietin und Satietin-D in gereinigter Form, dadurch **gekennzeichnet,** daß sie mittels einer Immunabsorbensmethode hergestellt sind.

2. Antisatietin und Antisatietin-D.

3. Antisatietin und Antisatietin-D, dadurch **gekennzeichnet,** daß sie die determinanten Gruppen des Satietins bzw. Satietin-D erkennen.

4. Verfahren zur Herstellung von Antisatietin beziehungsweise Antisatietin-D, dadurch **gekennzeichnet,** daß man Satietin beziehungsweise Satietin-D in üblicherweise für Immunisierungszwecke verwendete lebende Organismen parenteral einbringt und nach Ablauf der entsprechenden Zeit das Antisatienin beziehungsweise das Antisatietin-D aus dem Antiserum entsprechender Spezifität und Avidität in bekannter Weise abtrennt.

5. Der von Satietin und Antisatietin sowie der von Satietin-D und Antisatietin-D gebildete Immunkomplex.

6. Verfahren zur Herstellung des von Satietin mit Antisatietin gebildeten Immunkomplexes und des von Satietin-D mit Antisatietin-D gebildeten Immunkomplexes, dadurch **gekennzeichnet,** daß man Satietin und Antisatietin beziehungsweise Satietin-D und Antisatietin-D under Bedingungen kontaktiert, die eine Bindung ermöglichen.

7. Verfahren nach Anspruch 6, dadurch **gekennzeichnet,** daß man den Immunkomplex an einer festen Phase, vorzugsweise an einer aktivierten und äquilibrierten Gelsäule, bildet und dann das Satietin beziehungsweise Satietin-D mittels einer zur Spaltung von Immunkomplexen geeigneten Substanz selektiv desorbiert.

8. Verfahren zur Herstellung von selektiv appetitzügelnd wirkenden Wirkstoffen biologischen Ursprungs, nämlich Satietin und Satietin-D, in gereinigter Form, aus menschlichem oder tierischem Blutserum oder Blutplasma, dadurch **gekennzeichnet,** daß man menschliches oder tierisches Blutserum oder Blutplasma einer Ultrafiltration, einer Gelchromtographie, einer Behandlung mit Trichloressigsäure, einer Affinitätschromatographie und gewünschtenfalls der Behandlung mit einem proteolytischen Enzym, vorzugsweise Trypsin und Chymotrypsin, unterzieht, die Reinheit und Homogenität der erhaltenen Produktes mit einer immunchemischen Methode kontrolliert, zu dem immunchemisch homogenen Präparat Adjuvant gibt und das adjuvierte Präparat parenteral einem für die Zwecke der Immunisierung üblichen lebenden Organismus verabreicht und nach Ablauf der entsprechenden Zeit das polyclone monovalente Antisatietin und Antisatietin-D aus dem aus dem Blut des lebenden Organismus erhaltenen Antiserum geeigneter Spezifität und Avidität abtrennt und an eine geeignete feste Phase, vorzugsweise an ein aktiviertes und äquilibriertes Gel, bindet, dann durch die die Antikörper enthaltende Gelsäule ein äquilibriertes Ultrafiltrat, das die natürlichen, ein Molgewicht von weniger als 50 000 Dalton aufweisenden Bestandteile des menschlichen oder tierischen Blutserums oder Blutplasmas enthält, hindurchlaufen läßt und dann von der Säule aus dem von Satietin und Antisatietin beziehungsweise aus dem von Satietin-D und Antisatietin-D gebildeten Immunkomplex das Satietin beziehungsweise das Satietin-D desorbiert, die Eluate sammelt und gewünschtenfalls lyophilisert.

9. Verfahren nach Anspruch 9, dadurch **gekennzeichnet,** daß man von der den Immunkomplex gebunden enthaltenden Säule die restlichen natürlichen Bestandteile des menschlichen oder tierischen Blutserums oder Blutplasmas, die ein Molgewicht von unter 50 000 Dalton aufweisen, mit dem zum Äquilibrieren verwendeten Puffer auswäscht.

10. Verfahren nach Anspruch 8, dadurch **gekennzeichnet,** daß man den Immunkomplex an einer Sepharose-Gelsäule bildet.

11. Verfahren nach Anspruch 8, dadurch **gekennzeichnet,** daß man die Gelsäule mit Boratpuffer äquilibriert und mit Bromcyan aktiviert.

12. Verfahren nach Anspruch 8, dadurch **gekennzeichnet,** daß man das Satietin und das Satietin-D mit wässriger Ammoniumrhodanidlösung aus ihren an der Gelsäule gebundenen Immunkomplexen freisetzt.